# EUROPEAN PATENT APPLICATION

(11) **EP 2 918 261 A1**
(43) Date of publication of application: **16.09.2015**
(21) Application number: 15159047.8
(22) Date of filing: 13.03.2015
(51) Int. Cl.: A61K 8/36, A61Q 17/00, A61Q 19/10, A61K 31/20, A61K 33/30, A61K 33/34, A61P 31/00, A61K 36/752, A61K 36/53, A61K 36/54, A61K 36/61, A61P 35/00

(54) **FATTY ACID DERIVATIVES FOR TREATING INFECTIOUS DISEASES**

(30) Priority: 13.03.2014 FR 1452093
(71) Applicant: Imarko Research S.A., 4761 Petange (LU)
(72) Inventor: Rombi, Max, 18012 BORDIGHERA (IT)
(74) Representative: Regimbeau

(57) **Abstract**

The present invention relates to a fatty acid metal salt, where said fatty acid comprises 6 to 12 carbon atoms, said salt being a copper salt or a zinc salt, and compositions comprising same, for use in altering the lipid bilayer of cell membranes, and in particular for use as a medicine, as a cosmetic agent, as a medical device, as a food composition, as a nutraceutical or a food supplement.

## Description

The invention relates to the field of products intended to alter the lipid bilayer, and in particular their use as antibiotics. The products of the invention relate more particularly to fatty acid derivatives, including copper or zinc soaps, and to esters of caprylic acid and of lauric acid.

With the emergence of antibiotics, many fatal infections and many infectious diseases have been brought under control, and millions of lives have been saved. The great success of synthetic antibiotics, used against bacterial and fungal diseases, and of nucleoside analogs against viral diseases, has somewhat diverted researchers, and the pharmaceutical industry, from their efforts to develop new therapies in this field.

Unfortunately, the indiscriminate use of these synthetic antibiotics has also caused bacteria and viruses to develop resistance to the products used, making them particularly difficult to treat. Because new drug development has not taken this antibiotic resistance into account, these new resistant strains are causing numerous deaths in many hospitals.

Moreover, many synthetic drugs have severe side effects, which affect a large number of patients.

Despite these disadvantages, use of these synthetic antibiotics, in humans and in domesticated animals, remains inescapable in the absence of advantageous alternatives.

There is thus a need for other less problematic, natural antimicrobial products that kill pathogenic bacteria in a different way.

The Inventors have discovered that compounds of type copper or zinc soaps, in particular specific fatty acid copper salts or fatty acid zinc salts, are particularly useful as medicines, in particular in prophylactic or curative treatments of infectious diseases, in humans or in animals.

Such products can be used alone or in association with synthetic antibiotics, in order to limit the concentration of the latter.

The Inventors have thus discovered that certain fatty acid derivatives, due to their ability to alter the lipid bilayer of cell membranes, have in particular highly advantageous antibacterial properties.

The Inventors have in particular shown that fatty acid metal salts, and particularly fatty acid copper salts and zinc salts comprising typically 6 to 12 carbon atoms, may be advantageously used in particular as antibiotics. These compounds are particularly advantageous for use in therapeutic or prophylactic treatments of infectious diseases, in particular respiratory, digestive and urinary infectious diseases, candidiasis and mycosis. These compounds may in addition be used as anticancer agents, typically in combination with common treatments such as radiotherapy- and/or chemotherapy-based treatments.

The antibiotic properties of these compounds are considerable when fatty acids comprise 6 to 12 carbon atoms, and prove to be particularly advantageous when fatty acids comprise 6 to 10 carbon atoms, typically 6 to 8 carbon atoms or 8 to 10 carbon atoms.

These biocides of natural origin may be used particularly when strains have become resistant to conventional treatments. By their more constant antimicrobial and disinfectant action, these products may be of great help to failing antibiotics. A first object of the invention is a fatty acid metal salt, where said fatty acid comprises 6 to 12 carbon atoms, said metal salt being a copper salt or a zinc salt, or a composition comprising same, for use in altering the lipid bilayer of cell membranes.

A first object of the invention is thus a fatty acid metal salt, where said fatty acid comprises 6 to 12 carbon atoms and said metal salt is a copper salt or a zinc salt, or a composition comprising such a salt for use in altering the lipid bilayer of cell membranes.

In particular, said fatty acid metal salt or said composition may be used as a medicine, as a cosmetic agent, as a medical device, as a food composition, as a nutraceutical or a food supplement.

By **"fatty acid"** is meant in the sense of the invention an aliphatic-chain carboxylic acid. In the sense of the invention, a carboxylic acid is a compound comprising a carboxyl group (-COOH). In the sense of the invention, the term "aliphatic chain" refers to acyclic or cyclic, linear or branched, saturated or unsaturated hydrocarbon chains. Aliphatic chains according to the invention exclude aromatic hydrocarbon chains. Preferably, they will be acyclic, linear or branched, or saturated hydrocarbon chains.

By "fatty acid comprising 6 to 12 carbon atoms" is meant that the fatty acid comprises in total 6 to 12 carbon atoms, the fatty acid thus including the hydrocarbon chain and the COOH functional group. Thus, the hydrocarbon chain comprises only 5 to 11 carbon atoms. These fatty acids are called medium-chain fatty acids.

Fatty acids according to the invention may in particular have formula R-COOH with R representing a (C₅-C₁₁)alkyl or (C₅-C₁₁)alkenyl group.

By "alkyl" group is meant, in the sense of the present invention, a saturated linear hydrocarbon chain. A "(C₅-C₁₁)alkyl" group corresponds to an alkyl group as previously defined comprising 5 to 11 carbon atoms.

Advantageously, fatty acids having a saturated aliphatic chain, also called saturated fatty acids, according to the invention comprise, for example, caproic acid (CH₃-(CH₂)₄-COOH), enanthic acid (CH₃-(CH₂)₅-COOH), caprylic acid (CH₃-(CH₂)₆-COOH), pelargonic acid (CH₃-(CH₂)₇-COOH), capric acid (CH₃-(CH₂)₈-COOH), undecylic acid (CH₃-(CH₂)₉-COOH), lauric acid (CH₃-(CH₂)₁₀-COOH), and mixtures thereof.

Preferentially, the fatty acid according to the invention is a fatty acid having a saturated aliphatic chain.

The fatty acid according to the invention typically comprises 6 to 12 carbon atoms. Advantageously, the fatty acid according to the invention comprises 6 to 10 carbon atoms. In an advantageous manner, the fatty acid according to the invention comprises 6 to 8 carbon atoms or the fatty acid according to the invention comprises 8 to 10 carbon atoms.

In a particularly advantageous manner, the fatty acid according to the invention is a saturated fatty acid comprising 6 to 12 carbon atoms, i.e., a fatty acid of formula (C₅-C₁₁)alkyl-COOH. Thus, according to a preferred embodiment, the fatty acid according to the invention is selected from the list consisting of caproic acid, enanthic acid, caprylic acid, pelargonic acid, capric acid, undecylic acid, lauric acid, and mixtures thereof. Preferentially, the fatty acid according to invention is selected from the list consisting of caproic acid, caprylic acid, capric acid, lauric acid, and mixtures thereof.

In a very advantageous manner, the fatty acid according to the invention is a saturated fatty acid comprising 6 to 12 carbon atoms, i.e., a fatty acid of formula (C₅-C₉)alkyl-COOH. Thus, according to a preferred embodiment, the fatty acid according to the invention is selected from the list consisting of caproic acid, enanthic acid, caprylic acid, pelargonic acid, capric acid, and mixtures thereof. Preferentially, the fatty acid according to invention is selected from the list consisting of caproic acid, caprylic acid, capric acid, and mixtures thereof.

Preferably, the fatty acid according to the invention is caprylic acid.

By **"fatty acid metal salts"** is meant in the sense of the invention salts formed with the carboxyl functional group of the fatty acid to give carboxylates of metal ions, in particular copper carboxylates and zinc carboxylates.

The fatty acid metal salts according to the invention may be obtained by the well-known chemical reaction called saponification. The fatty acid metal salts of the invention may in particular be obtained by alkaline saponification of the above-mentioned fatty acids of the invention, or a mixture of fats comprising same, with a strong base preferably selected from copper hydroxide, Cu(OH)₂, or zinc hydroxide, Zn(OH)₂, in alkaline medium, typically at a temperature between 80 °C and 250 °C.

Advantageously in the sense of the invention, fatty acid copper salts comprise caproic acid copper salt, or copper caproate; enanthic acid copper salt, or copper enanthate; caprylic acid copper salt, or copper caprylate; pelargonic acid copper salt, or copper pelargonate; capric acid copper salt, or copper caprate; undecylic acid copper salt, or copper undecylate; lauric acid copper salt, or copper laurate; and mixtures thereof.

Advantageously in the sense of the invention, fatty acid zinc salts comprise caproic acid zinc salt, or zinc caproate; enanthic acid zinc salt, or zinc enanthate; caprylic acid zinc salt, or zinc caprylate; pelargonic acid zinc salt, or zinc pelargonate; capric acid zinc salt, or zinc caprate; undecylic acid zinc salt, or zinc undecylate; lauric acid zinc salt, or zinc laurate; and mixtures thereof.

Preferentially, said metal salt is used for therapeutic or prophylactic treatment of at least one infectious disease in a subject, preferably selected from the list consisting of respiratory infectious diseases, digestive infectious diseases, urinary infectious diseases, candidiasis and mycosis.

These fatty acid soaps are also active against viral diseases when the virus have a lipid envelope, such as influenza virus or Ebola virus.

In reference to a molecule, by **"antibiotic"** is meant in the sense of the invention the ability of said molecule to inhibit the growth of bacteria or to destroy bacteria.

By **"prophylactic treatment"** is meant in the sense of the present invention treatment aimed at decreasing the risks of developing pathology.

By **"infectious disease"** is meant in the sense of the present invention a disease caused by the transmission of a microorganism. In the context of the invention, the term "microorganism" refers equally to viruses, in particular virus which have a lipid envelope (such as influenza virus, for example), bacteria, parasites, and fungi.

By **"respiratory infectious disease"** is meant in the sense of the present invention an infectious disease concerning the respiratory system. The respiratory system in the sense of the invention comprises the upper respiratory tract and the lower respiratory tract. The upper respiratory tract is composed of the nose, the pharynx comprising the nasopharynx, the oropharynx and the laryngopharynx, and associated structures, which comprise the middle ear and the auditory tube. The lower respiratory tract comprises the larynx, the trachea, the bronchi and the pulmonary alveoli.

In the sense of the invention, the term "respiratory infectious disease" refers, among other things, to laryngitis (which can be caused for example in humans by *Streptococcus pneumoniae* or S. *pyogenes,* or by a virus), tonsillitis (which can be caused by the same microorganisms responsible for laryngitis), sinusitis (which can be caused for example in humans by S. *pneumoniae* or *Haemophilus influenzae),* epiglottitis (H. *influenzae* type b), streptococcal pharyngitis, or streptococcal angina, scarlet fever (caused in humans by *Staphylococcus aureus*)*,* diphtheria (caused in humans by *Corynebacterium diphtheriae*)*,* otitis media (which can be due to S. *pneumoniae, H. influenzae, Moraxella catarrhalis, S. pyogenes* and S. *aureus* microorganisms), colds (due to *Rhinovirus* and *Coronavirus* viruses), bronchitis, bronchiolitis, pneumonia, pertussis (due in humans to *B. pertussis*), tuberculosis (caused in humans by *Mycobacterium tuberculosis*), bacterial pneumonia and in particular pneumococcal pneumonia (caused by S. *pneumoniae*), pneumonia caused by *Haemophilus influenzae,* mycoplasmal pneumonia (caused by *Mycoplasma pneumoniae*)*,* chlamydial pneumonia (due to an organism of the genus *Chlamydia,* most often *Chiamydia pneumoniae*), legionellosis (due to *Legionella pneumophila*), psittacosis (caused by *Chlamydia psittaci*), influenza (due to viruses of the genus *Influenzavirus*), histoplasmosis (due to *Histoplasma capsulatum*)*,* coccidioidomycosis (caused by *Coccidioides immitis*), pneumocystis pneumonia (due to *Pneumocystis carinii*), blastomycosis (caused by *Blastomyces dermatitidis*), aspergillosis (due to *Aspergillus fumigatus*).

By **"digestive infectious disease"** is meant in the sense of the present invention an infectious disease concerning the digestive system. The digestive system, in the sense of the invention, comprises the esophagus, the stomach and the intestines (the small intestine and the large intestine, also called the colon).

In the sense of the invention, the term "digestive infectious disease" refers, among other things, to gastroenteritis or enteric infection.

By **"urinary infectious disease"** is meant in the sense of the present invention an infectious disease concerning the urinary system. The urinary system, in the sense of the invention, comprises both kidneys, both ureters, the bladder and the urethra.

In the sense of the invention, the term "urinary infectious disease" refers, among other things, to cystitis due to infection (bladder infection in women most often due to *Escherichia coli*) and pyelonephritis (upper urinary tract infection, thus concerning the renal pelvis (pyelitis) and renal parenchyma (nephritis), most often resulting from infection by *Escherichia coli*)*.*

By **"candidiasis" or "mycosis"** is meant in the sense of the present invention fungal infection caused by yeasts of the genus *Candida,* preferably *Candida albican,* and all known mycosis.

In the sense of the invention, the term "candidiasis" refers, among other things, to mucocutaneous candidiases and systemic candidiases.

Mucocutaneous candidiases comprise in particular oral candidiases such as thrush in nursing babies or black hairy tongue, vulvovaginitis and urethritis.

The antibiotic properties of fatty acid metal salts or compositions comprising same according to the invention make them in addition particularly suited to the prevention and treatment of skin infections such as for example mycoses or infections commonly related to skin diseases, such as psoriasis or keratoses.

Furthermore, because of their detergent properties, products of the invention are particularly suited to the prevention and treatment of skin disorders or ailments involving hypertrophy of the corneal layers of the epidermis, such as psoriasis or keratoses.

By **"subject"** is preferably meant in the sense of the invention an animal or a human.

Moreover, the Inventors discovered that the fatty acid metal salts of the invention, i.e., the copper salts or zinc salts described above, may be advantageously used in anticancer treatments, for example as adjuvants for common treatments. Fatty acid metal salts of the invention are particularly effective in combination with at least one radiotherapy- and/or chemotherapy-based treatment. Indeed, fatty acid metal salts of the invention, by their detergent action typically on the lipid bilayer of cell membranes, make it possible to destroy cells damaged by radiotherapy or chemotherapy, while decreasing the side effects generally observed when these therapies are given alone.

Use of fatty acid metal salts of the invention thus allows more complete action on tumors and improves the patient's well-being by limiting the drastic effects of current protocols.

Thus, preferentially, said metal salt is useful for therapeutic treatment of cancer, preferably in combination with at least one radiotherapy- or chemotherapy-based treatment.

It may for example be used in the form of capsules or tablets to be taken at the same time as the basic treatment.

The Inventors have further shown that metal salts of the invention, namely fatty acid copper salts and fatty acid zinc salts, may be advantageously mixed with monoglycerides.

The invention thus also relates to the composition of the invention, comprising monoglycerides.

By **"monoglyceride"** or monoacylglycerol (MAG) is meant in the sense of the invention esters formed of a fatty acid residue combined with a glycerol residue.

Advantageously in the sense of the invention, monoglycerides comprise glycerol monocaproate, glycerol monoethanate, glycerol monocaprylate, glycerol monopelargonate, glycerol monocaprate, glycerol monoundecylate, glycerol monolaurate, and mixtures thereof.

The Inventors have further discovered that the properties of metal salts of the invention, namely fatty acid copper salts and fatty acid zinc salts, are increased in a surprising manner when they are associated with at least one plant essential oil. The association of these two types of molecules makes it possible to obtain a strengthened effect on antibiotic activity (activity on a broader spectrum of microorganisms) that is particularly strong when essential oils of thyme, ravintsara, laurel, for example bay laurel, lime, eucalyptus, cinnamon, tea tree (*Melaeuca alternifolia*) are used.

The invention thus also relates to the composition of the invention, comprising at least one essential oil, preferably selected from thyme essential oil, ravintsara essential oil, laurel essential oil, for example bay laurel essential oil, lime essential oil, eucalyptus essential oil, cinnamon essential oil, tea tree (*Melaleuca alternifolia*) essential oil, and mixtures thereof.

These properties are particularly advantageous when the composition of the invention comprises both:
- at least one monoglyceride, preferably selected from glycerol monocaproate, glycerol monoethanate, glycerol monocaprylate, glycerol monopelargonate, glycerol monocaprate, glycerol monoundecylate, glycerol monolaurate and mixtures thereof, and
- at least one essential oil, preferably selected from thyme essential oil, ravintsara essential oil, laurel essential oil, for example bay laurel essential oil, lime essential oil, eucalyptus essential oil, cinnamon essential oil, tea tree (*Melaieuca alternifolia*) essential oil and mixtures thereof.

The composition of the invention comprises typically between 10 and 60% by weight, preferentially between 50 and 60%, more preferentially between 54 and 59% by weight, of fatty acid metal salts, with respect to the total weight of the composition.

The composition of the invention comprises typically between 30 and 80% by weight, preferentially between 30 and 40%, more preferentially between 36 and 39% by weight, of monoglyceride, with respect to the total weight of the composition.

The composition of the invention comprises typically between 1 and 10% by weight, preferentially between 2.3 and 10%, more preferentially between 2.31 and 9.24% by weight, of essential oil, with respect to the total weight of the composition.

According to an embodiment, the composition of the invention is in a form suitable for topical administration, in particular dermatological. It may be advantageously provided in all pharmaceutical forms usually used for topical application, in particular in the form of an aqueous, hydroalcoolic or oil solution, an oil-in-water or water-in-oil or multiple emulsion, an aqueous or oil gel, a liquid anhydrous product, paste or solid, an oil-in-aqueous phase dispersion using spherical particles (nanospheres, nanocapsules, lipid vesicles), a transdermal device or in any other form for topical application.

This composition may be relatively fluid and have the appearance of a cream, an ointment, a milk, a lotion, a serum, a paste, a foam or a gel. Optionally, it may be applied to the skin in aerosol form. It may also be provided in solid form, for example stick form. It may also be applied by means of a patch.

In the context of topical application, this composition may further comprise a pharmaceutically acceptable carrier or excipient, in particular a pharmaceutically acceptable carrier or excipient for topical administration, in particular dermatologically acceptable, i.e., a support compatible with the skin. Creams containing all these products are highly advantageous for treating psoriasis and keratoses.

According to another embodiment of the invention, the composition is in a form suitable for systemic administration, in particular enteral. In the sense of the invention, the enteral route comprises the perlingual, oral or digestive, and rectal routes.

It may be advantageously provided in all pharmaceutical forms usually used for enteral administration, in particular in oral liquid forms such as syrups, oral dispersions, drops; in paste forms, optionally in sealed capsules or soft gelatin capsules; in dry pulverulent forms such as powders or granules, optionally in hard capsules or gelatin capsules; compact dry forms such as tablets; and suppositories.

The composition of the invention is also very efficient in preventive or curative treatments of mycosis diseases affecting grapevine.

The invention further relates to nontherapeutic use of at least one fatty acid metal salt, where said fatty acid comprises 6 to 12 carbon atoms, said salt being a copper salt or a zinc salt, or a composition comprising same as a cosmetic agent, as a medical device, as a food composition, as a nutraceutical or a food supplement.

In the sense of the present invention, a medical device (MD) is defined as any instrument, apparatus, equipment, material or other article, used alone or in combination, including software required for the proper functioning thereof, intended by the manufacturer to be used in humans for the purposes of:
- diagnosing, preventing, controlling, treating or alleviating a disease,
- diagnosing, controlling, treating, alleviating or compensating for an injury or a handicap,
- studying or replacing or modifying the anatomy or a physiological process,
- controlling design,
and whose principal action wanted in or on the human body is not obtained by pharmacological or immunological means nor by metabolism, but whose function may be assisted by such means.

By nontherapeutic use is meant in the sense of the invention that the intended use does not allow and/or is not intended to prevent or treat a pathological condition. The term "prevention" refers to measures taken to reduce the risk of developing a pathological condition.

The products of the invention may be advantageously used in nontherapeutic applications, such as, for example, in compositions for disinfecting the skin, such compositions being able to make it possible in particular to avoid development of bacteria and thus to limit development of odor or to limit superinfection of skin lesions.

Thus, the invention relates, for example, to nontherapeutic products for personal hygiene, such as in particular bath gels for the skin, shampoos, products intended for cleaning the mucous membranes (such as, for example, solutions intended for cleaning the skin, the auricle or the nasal cavities), deodorant products. The application further relates to auxiliary products for preventing disorders associated with an imbalance of commensal microbial flora, such as imbalances of cutaneous, respiratory, genital or digestive commensal flora.

Lastly, the invention relates to a method of cosmetic care of the skin, hair, nails or mucous membranes, in order to improve the condition or the appearance thereof, consisting in administering at least one fatty acid metal salt or composition comprising same, where said fatty acid comprises 6 to 12 carbon atoms, said salt being a copper salt or a zinc salt.

The Inventors have further demonstrated the surprising efficacy of particular esters of caprylic acid and/or of lauric acid to alter the lipid bilayer of cell membranes, in particular, as a medicine, as a cosmetic agent, as a medical device, as a food composition, as a nutraceutical or a food supplement.

These esters comprise in particular thymol caprylate, thymol laurate, carvacrol caprylate, carvacrol laurate, geraniol caprylate, geraniol laurate and mixtures thereof. The Inventors have thus shown that esters of caprylic acid and/or of lauric acid and phenols and/or alcohols typically contained in plant essential oils can be used to alter the lipid bilayer of cell membranes. These properties make them very good antibiotic products able to be used, for example, in the curative or prophylactic treatment of infectious diseases. Another object of the invention is thus an ester of caprylic and/or of lauric acid, said ester being selected from thymol caprylate, thymol laurate, carvacrol caprylate, carvacrol laurate, geraniol caprylate, geraniol laurate and mixtures thereof, for use as a medicine, advantageously in the therapeutic or prophylactic treatment of at least one infectious disease or in the treatment of cancer.

The ester of the invention may be easily obtained by esterification and/or transesterification reaction, well known to the skilled person. Typically, the esterification reaction consists in reacting, respectively, in the presence or absence of catalyst, caprylic acid with an alcohol or a phenol or an acyl chloride, preferably with capryloyl chloride, in order to obtain an ester according to the invention. The transesterification reaction consists in reacting a fat (to be specific an ester of caprylic acid) with an alcohol in order to obtain a fatty acid ester according to the invention. The alcohol and the phenol taking part in the reaction to form the desired ester are selected in particular from thymol, carvacrol and geraniol.

The Inventors have further discovered that esters of thymol caprylate, thymol laurate, carvacrol caprylate, carvacrol laurate, geraniol caprylate, geraniol laurate, may be formed directly in the human body, from caprylic acid and/or lauric acid and at least one essential oil. The Inventors have thus developed products, so-called kits-of-parts, comprising these two elements, which may be administered simultaneously or separately.

Thus, the invention also relates to products containing:
- caprylic acid or a derivative thereof and/or lauric acid or a derivative thereof,
- at least one essential oil selected from thyme essential oil, ravintsara essential oil, laurel essential oil, for example bay laurel essential oil, lime essential oil, eucalyptus essential oil, cinnamon essential oil, tea tree (*Melaieuca alternifolia*) essential oil and mixtures thereof,
as combination product for simultaneous, separate or sequential use, for use in altering the lipid bilayer of cell membranes, for use as a medicine, as a cosmetic agent, as a medical device, as a food composition, as a nutraceutical or a food supplement, advantageously in the therapeutic or prophylactic treatment of at least one infectious disease or in the treatment of cancer.

By **"derivative"** of caprylic acid or of lauric acid is meant in particular an ester of caprylic acid or of lauric acid, such as an alkyl ester, in particular a (C₆-C₁₂)alkyl ester; a metal salt of caprylic acid or of lauric acid, such as a sodium, potassium, calcium, magnesium, silver, copper or zinc salt; a monoglycerol of caprylic acid or of lauric acid; or a mixture thereof.

The diseases and disorders targeted by the ester of the invention, by compositions comprising same or by products comprising same, and the subjects able to benefit from such treatments, are as defined above in the present application.
The compositions or products of the invention may further comprise additional active products, such as those described in the present application.

These compositions or products may be in a form suitable for systemic administration, in particular enteral or parenteral, or for topical administration, in particular dermatological, as defined in the present application.

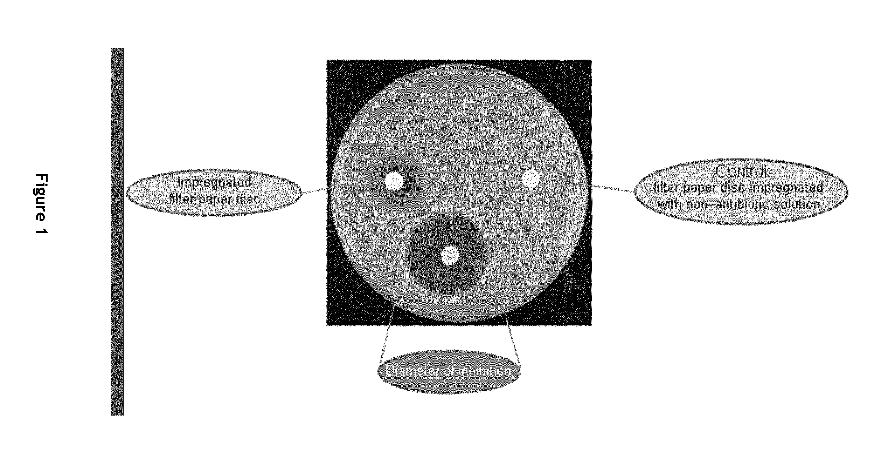

Caption for Figure 1: Illustration of the diffusion method in agar medium: photograph of a petri dish with zone of inhibition.

### Examples

The antibiotic activity of zinc and copper soaps (salts of copper caprylate, caprate, caproate and laurate; and salts of zinc caprylate, caprate, caproate and laurate) was measured according to the diffusion method in agar medium, the protocol of which is detailed below.

This method consists in depositing on inoculated agar culture medium sterile absorbent paper discs impregnated with the antibiotic solution to be examined. The activity of the antibiotic substance is measured by the diameter of the zone of inhibition, i.e., the diameter of the surface on which the microorganism did not grow.

The activity of an antibiotic is estimated by comparing the inhibition of growth of sensitive microorganisms caused respectively by known concentrations of the antibiotic to be examined and of a reference substance.

### Protocol:

### The diluents and culture media used are:

- 0.85% physiological saline (AES, reference PHYW-AEB110389),
- buffered peptone water (bioMérieux, reference 42111),
- Sabouraud glucose agar with chloramphenicol (bioMérieux, reference 42620),
- Neutral MRS agar pH = 6.4 (AES, reference AEB621756N),
- Trypcase soy agar (bioMérieux, reference 41466),
- Cetrimide agar (bioMérieux, reference 43565).

The agar culture medium is poured into a petri dish. Once solidified, the surface of the agar is inoculated in a layer with the selected microorganism. Then filter paper discs, 6.6 mm in diameter, impregnated with the various solutions to be tested are placed on the surface. The petri dishes are left at room temperature for about 1 hour to allow the substances to diffuse in the agar.

The cultures are then incubated at 32.5 °C ± 2.5 °C. The agar media and the incubation conditions are suited to the inoculated microorganism.

The antibiotic activity of the solution is expressed by a zone of inhibition, i.e., a circle around the filter paper within which the inoculated microorganism did not grow.

The diameter of the zone of inhibition is a function of the concentration, diffusion and activity of the tested substance placed on the filter.

### Results:

First, we measured by this method the sensitivity, to our anti-infectious soaps, of 10 widely known bacteria-Gram-positive and Gram-negative bacteria-and also a yeast.

All these bacteria were sensitive to copper soaps, with a few differences depending on their Gram-positive or Gram-negative classification.

The Gram-positive bacteria were more resistant and slightly less sensitive.

The *Candida* were very sensitive, as were the Gram-positive bacteria.

The same tests were carried out with zinc soaps.

Copper soaps appear to be more effective than zinc soaps in fighting infection.

Various fatty acid copper or zinc salts were tested on various strains of bacteria or yeast. The results obtained are presented in table 1 below.

**Table 1**

| Strains tested | Copper octanoate | Zinc octanoate | Copper octanoate solution (496.0 mg in 2.0 ml of DMSO) |
|---|---|---|---|
| *Saccharomyces cerevisiae* | +++ | ++ | ++ |
| *Lactobacillus acidophilus* | +++ | + | +++ |
| *Lactobacillus plantarum* | +++ | + | ++ |
| *Lactobacillus paracasei* | ++++ | ++ | nd |
| *Bacillus coagulans* (spores) | ++++ | ++ | nd |

| | | | |
|---|---|---|---|
| nd = not determined DMSO = dimethyl sulfoxide | | | |

Various monoglycerides were also tested on various strains of bacteria or yeast. The results obtained are presented in table 2 below.
Strains tested Glyceryl caprylate Glycerol Caprylauric monolaurate *Saccharomyces* inhibition no inhibition inhibition *cerevisiae Lactobacillus* inhibition inhibition inhibition inhibition inhibition inhibition *acidophilus Lactobacillus* inhibition no inhibition weak inhibition *plantarum Bifidobacterium* inhibition no inhibition inhibition *Lactobacillus* inhibition no inhibition inhibition inhibition no inhibition inhibition *paracasei Bacillus coagulans* inhibition inhibition inhibition (spores) inhibition inhibition inhibition Table 2
Glyceryl caprylate: Capmul MCM C-8 from Unipex
Glycerol monolaurate: marketed by Oleon
Caprylauric: 60:40 mixture of monoglycerol caprylate and monoglycerol laurate

Thymol caprylate in solution in dimethyl sulfoxide (200 mg in 2 ml of DMSO) was also tested on various strains of bacteria or yeast. The results obtained are presented in table 3 below.

**Table 3**

| Strains tested | Diameter of inhibition (in mm) | | |
|---|---|---|---|
| | Sterile distilled water | DMSO | Thymol caprylate in solution in DMSO |
| | | | |
| *Saccharomyces cerevisiae* | 6.6 | 6.6 | 7.28 |
| *Lactobacillus acidophilus* | 6.6 | 6.6 | 8.2 |
| *Lactobacillus plantarum* | 6.6 | 6.6 | 6.9 |
| *Bifidobacterium* | 6.6 | 6.6 | 9 |
| *Lactobacillus paracasei* | 6.6 | 6.6 | 7.75 |
| *Bacillus coagulans* (spores) | 6.6 | 6.6 | 8.5 |
| *Pseudomonas putida* | 6.6 | 6.6 | 7.86 |

Lastly, the following two caprylic acid derivatives-based and lauric acid derivatives-based mixtures were also tested on various strains of bacteria and yeast:
- caprylaurate: comprising monoglycerol caprylate (54.46%), monoglycerol laurate (36.30%) and essential oils (9.24%) (namely, ravintsara essential oil (2.31%), thyme essential oil (2.31%), noble laurel essential oil (2.31%), lime essential oil (2.31%));
- caprylaurate/thyme: comprising sodium caprylate (40%), monoglycerol laurate (40%) and thyme essential oil (6%), and excipients (14%) (namely, silica (12.5%), magnesium stearate (1.5%)).

Both mixtures were tested in solution in a mixture of DMSO and sterile water (200 mg of mixture in 2 ml of DMSO/sterile water (1:1)). The results obtained are presented in table 4 below.

**Table 4**

| Strains tested | Diameter of inhibition (in mm) | | | |
|---|---|---|---|---|
| | Sterile water | DMSO | Caprylaurate in solution in DMSO/sterile water | Caprylaurate/thyme in solution in DMSO/sterile water |
| *Saccharomyces cerevisiae* | 6.6 | 6.6 | 10.7 | 15.8 |
| *Lactobacillus acidophilus* | 6.6 | 6.6 | 9 | 13.5 |
| *Bifidobacterium* | 6.6 | 6.6 | 14.4 | 14.1 |
| *Lactobacillus paracasei* | 6.6 | 6.6 | 10.4 | 16.7 |
| *Bacillus coagulans* (spores) | 6.6 | 6.6 | 15 | 18.4 |
| *Pseudomonas putida* | 6.6 | 6.6 | 10.6 | 6.6 |
| *Pseudomonas fluorescens* | 6.6 | 6.6 | 9.6 | 6.6 |

## Claims

1. Fatty acid metal salt, where said fatty acid comprises 6 to 12 carbon atoms, said salt being a copper salt or a zinc salt, or composition comprising same, for use in altering the lipid bilayer of cell membranes.

2. Metal salt or composition according to claim 1, for use as a medicine, as a cosmetic agent, as a medical device, as a food composition, as a nutraceutical or a food supplement.

3. Metal salt or composition according to claim 1 or 2, for use in the therapeutic or prophylactic treatment of at least one infectious disease in a subject, preferably selected from the list consisting of respiratory infectious diseases, digestive infectious diseases, urinary infectious diseases and candidiases.

4. Metal salt or composition according to any one of claims 1 to 3, for use in the treatment of cancer, preferably in combination with at least one radiotherapy- and/or chemotherapy-based treatment.

5. Metal salt or composition for use according to any one of claims 1 to 4, **characterized in that** said fatty acid is selected from caproic acid, caprylic acid, capric acid, lauric acid, and mixtures thereof, advantageously said fatty acid being caprylic acid.

6. Composition for use according to any one of claims 1 to 5, **characterized in that** it further comprises at least one essential oil, preferably selected from thyme essential oil, ravintsara essential oil, laurel essential oil, for example bay laurel essential oil, lime essential oil, eucalyptus essential oil, cinnamon essential oil, tea tree (*Melaieuca alternifolia*) essential oil, and mixtures thereof.

7. Nontherapeutic use of at least one fatty acid metal salt, where said fatty acid comprises 6 to 12 carbon atoms, said salt being a copper salt or a zinc salt, or a composition comprising said metal salt, as a cosmetic agent, as a medical device, as a food composition, as a nutraceutical or a food supplement, preferentially nontherapeutic products for personal hygiene, such as in particular bath gels for the skin, shampoos, products intended for cleaning the mucous membranes (such as, for example, solutions intended for cleaning the skin, the auricle or the nasal cavities), deodorant products, and secondary products for preventing disorders associated with an imbalance of commensal microbial flora, such as imbalances of cutaneous, respiratory, genital or digestive commensal flora.

8. Ester of caprylic and/or of lauric acid selected from thymol caprylate, thymol laurate, carvacrol caprylate, carvacrol laurate, geraniol caprylate, geraniol laurate, and mixtures thereof, for use in altering the lipid bilayer of cell membranes.

9. Ester of caprylic and/or of lauric acid according to claim 8, for use as a medicine, as a cosmetic agent, as a medical device, as a food composition, as a nutraceutical or a food supplement.

10. Ester according to claim 9, for use in the therapeutic or prophylactic treatment of at least one infectious disease or in the treatment of cancer.

11. Nontherapeutic use of at least one ester of caprylic and/or of lauric acid, said ester being selected from thymol caprylate, thymol laurate, carvacrol caprylate, carvacrol laurate, geraniol caprylate, geraniol laurate and mixtures thereof, as a cosmetic agent, as a medical device, as a food composition, as a nutraceutical or a food supplement.

12. Product containing:
- caprylic acid or a derivative thereof and/or lauric acid or a derivative thereof, and
- at least one essential oil selected from thyme essential oil, ravintsara essential oil, laurel essential oil, for example bay laurel essential oil, lime essential oil, eucalyptus essential oil, cinnamon essential oil, tea tree (*Melaieuca alternifolia*) essential oil, and mixtures thereof,
as combination product for simultaneous, separate or sequential use, for use in altering the lipid bilayer of cell membranes, for use as a medicine, as a cosmetic agent, as a medical device, as a food composition, as a nutraceutical or a food supplement, advantageously in the therapeutic or prophylactic treatment of at least one infectious disease or in the treatment of cancer.
